# EUROPÄISCHE PATENTSCHRIFT

(11) **EP 0 352 640 B1**
(45) Veröffentlichungstag und Bekanntmachung des Hinweises auf die Patenterteilung: **16.06.1993**
(21) Anmeldenummer: 89113380.3
(22) Anmeldetag: 21.07.1989
(51) Int. Cl.: C07D 207/333, A61K 31/40, C07D 209/12, C07D 295/08, A61K 31/495, C07D 231/12, A61K 31/415, C07D 333/22, A61K 31/38

(54) **p-Hydroxiphenon-Derivate und diese enthaltende Arzneimittel**
p-Hydroxyphenone derivatives and pharmaceutical compositions thereof
Dérivés de p-hydroxyphénone et médicaments les contenant

(30) Priorität: 28.07.1988 DE 3825561
(43) Veröffentlichungstag der Anmeldung: 31.01.1990
(73) Patentinhaber: BASF Aktiengesellschaft, 67063 Ludwigshafen (DE)
(72) Erfinder: Lubisch, Wilfried, Dr., D-6800 Mannheim 1 (DE); von Philipsborn, Gerda, Dr., D-6940 Weinheim (DE)

(56) Entgegenhaltungen:
- EP-A- 0 152 556
- EP-A- 0 201 400
- FR-A- 2 348 189
- JOURNAL OF THE AMERICAN PHARMACEUTICAL ASSOCIATION, Band 47, Nr. 9, September 1958, Seiten 640-645, Washington, US; G.V. ROSSI et al.: "An evaluation of the pharmacologic activity of a new series of chalcone derivatives"

## Beschreibung

Die Erfindung betrifft p-Hydroxyphenon-Derivate, sie enthaltende Arzneimittel und deren Verwendung zur Herstellung von Antiarrhythmika der Klasse III nach Vaughan-Williams.

Die Antiarrhythmika lassen sich aufgrund der Klassifizierung von Vaughan-Williams in 4 Gruppen einteilen:
I. Natrium-Antagonisten,
II. Adrenerge β-Rezeptorblocker
III. Kalium-Kanal-Hemmstoffe und
IV. Calcium-Antagonisten

Antiarrhythmika der Klasse III weisen häufig den therapeutischen Vorteil auf, gegen sonst therapieresistente Arrhythmien zu wirken, insbesondere in der klinischen Therapie von reentry-Arrhythmien. Dies wurde sowohl beim Amiodaron (Cirlulation, 68(1), 88-94 (1983) als auch beim D-Sotalol (Am Heart J. 109, 949-958 (1985); J. Clin. Pharmacol. 27 (9) 708 (1987)) beschrieben.

p-Hydroxyphenone wurden bereits mit unterschiedlicher physiologischer Wirkung beschrieben: Spasmolytika (DE 26 16 484; DE 1 174 311, Arch. Pharm. 1966, 299): Ulcustherapeutika (JP-OS 52/078 858; JP-OS 52/078 856; JP-OS 51/100 050); Amöbizide (FR. 5003 M); Vasodilatantien (JP-AS 27 177/65; GB 1 022 648; US 3 407 233; DE 2 062 129; JP-AS 40/06 903; JP-AS 40/06 904; JP-AS 74/021 125; Ca-Antagonisten (EP 201 400; EP 209 435).

Die in dieser Erfindung beanspruchten p-Hydroxyphenon-Derivate stellen dagegen überraschenderweise Antiarrhythmika der Klasse III dar.

Die Erfindung bezieht sich auf p-Hydroxyphenon-Derivate der Formel I 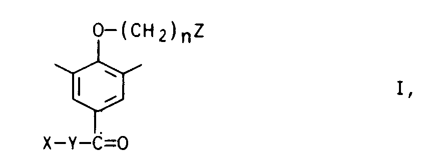
worin
- X: den Rest 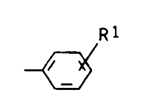 mit R¹ = F, Cl, Br oder Trifluormethyl oder einen Heteroarylrest der Struktur 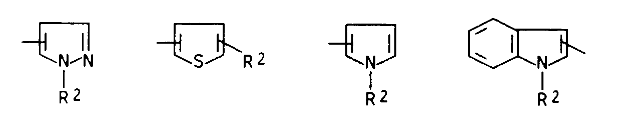 mit R² = C₁-C₄-Alkyl,
- Y: ein Brückenglied der Formel -CH=CH-, -CH₂CH₂- oder -CH₂-,
- n: eine der Zahlen 2, 3 oder 4 und
- Z: eine Aminogruppe der Formel -NR²R³ oder den N-Pyrrolidinyl- oder N-Piperidylrest bedeutet, wobei R² und R³ unabhängig voneinander C₁-C₄-Alkyl bedeuten, sowie deren physiologisch verträglichen Salze.

Die erfindungsgemäßen Verbindungen können beispielsweise nach folgenden Reaktionsschemata A und B hergestellt werden:

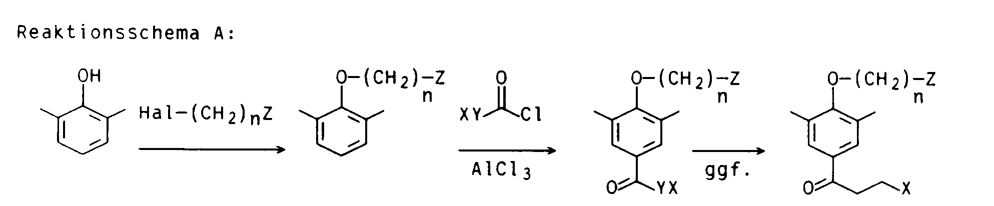

2,6-Dimethylphenol wird in üblicher Weise mit einem Halogenalkylamin alkyliert, wonach eine Friedel-Crafts-Reaktion mit einem Säurechlorid X-Y-COCl zu dem Hydroxyphenon führt. Falls Y die Gruppe -CH=CH- bedeutet, führt eine katalytische Hydrierung unter üblichen Bedingungen zu den Propionphenonen.

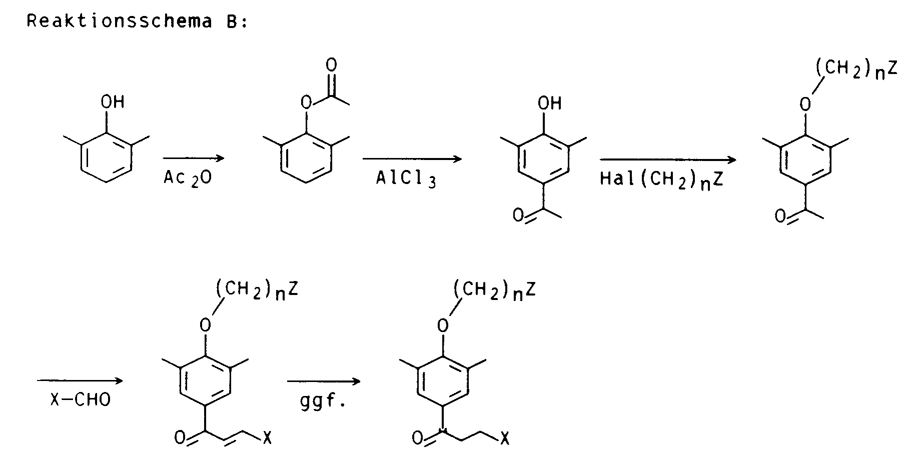

2,6-Dimethylphenol wird mit Essigsäureanhydrid in üblicher Weise verestert. Durch Fries-Verschiebung in einer AlCl₃-Schmelze erfolgt die Phenol-Acylierung. Die Kondensation mit einem Aldehyd X-CHO erfolgt unter basischen Bedingungen, üblicherweise mit NaOH. Die letzten beiden Reaktionsschritte können auch in umgekehrter Reihenfolge durchgeführt werden. Eine anschließende Hydrierung analog dem Schema A führt zu dem Propiophenon.

Gegebenenfalls werden die so erhaltenen Propiophenonderivate in das Säureadditionssalz einer physiologisch verträglichen Säure übergeführt. Eine Zusammenstellung üblicher physiologisch verträglicher Säuren kann aus Fortschritte der Arzneimittelforschung, 1966, Birkhäuser Verlag, Bd. 10, S. 224 bis 285, Deutschland, Schweiz, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der p-Hydroxiphenonderivate der Formel I durch Auflösen der freien Basen in einer wäßrigen Säurelösung hergestellt werden.

Die Erfindung betrifft weiter therapeutische Mittel zur topischen und vor allem systemischen Anwendung, die eine Verbindung der Formel I neben üblichen Trägerstoffen und/oder sonstigen galenischen Hilfsmitteln als Wirkstoff enthalten, und die Verwendung einer Verbindung der Formel I zur Herstellung eines Arzneimittels, insbesondere eines Antiarrhythmikums.

Die neuen Verbindungen besitzen, wie die folgenden Versuchsergebnisse zeigen, eine gute antiarrhythmische Klasse-III-Wirkung:
Als Versuchstiere dienen männliche und weibliche Pirbright-white-Meerschweinchen im Gewicht von 300 bis 500 g. Die Narkose erfolgt mit 1,5 g/kg Urethan i.p.. Die Substanzen werden intravenös appliziert. Zur Messung der EKG-Leitungszeiten und der Herzfrequenz wird die II. Extremitätenableitung registriert. Meßgrößen sind die QT- und die PQ-Strecken und die Herzfrequenz. Pro Dosis werden 4 bis 6 Tiere eingesetzt. Kriterium für die Klasse-III-Wirkung ist eine Zunahme der QT-Dauer im Vergleich zu den Werten vor Substanzgabe. PQ-Zunahme und starke Herzfrequenzabnahme dienen als Ausschlußkriterien. Aus der linearen Beziehung zwischen log Dosis (mg/kg) der Substanz und der relativen Verlängerung der QT-Dauer (in %) wird die ED 20% berechnet.

**Tabelle 1**

| Antiarrhythmische Klasse-III-Wirkung beim Meerschweinchen nach intravenöser Applikation. | |
|---|---|
| Beispiel | Verlängerung der QT-Dauer ED 20% [mg/kg] |
| 2 | 1,1 |
| 4 | 1,2 |
| 5 | 1,0 |
| D-Sotalol | 3,6 |

Die neuen Substanzen eignen sich daher zur Behandlung sonst therapieresistenter Arrhythmien, insbesondere beseitigen sie ventrikuläre Tachykardien, die nach Myokard-Infarkt auftreten und auf einem "re-entry"-Mechanismus beruhen (Lit. Cardiac Arrhythmia Ed. P. Brugada, H.J.J. Wellens, Futura Publishing & Co., Mount Kisko, New York 1987).

Die Herstellung der therapeutischen Mittel oder Zubereitungen erfolgt mit den üblichen flüssigen oder festen Trägerstoffen oder Verdünnungsmitteln und den üblicherweise verwendeten pharmazeutisch-technischen Hilfsstoffen, entsprechend der gewünschten Applikationsart und mit einer zur Anwendung geeigneten Dosierung, in üblicher Weise, beispielsweise durch Vermischen des Wirkstoffes mit den an sich in solchen Präparaten üblichen festen und flüssigen Träger- und Hilfsstoffen.

Die Mittel können peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,0001 bis 1%iger Konzentration, bevorzugt in 0,001 bis 0,1%iger Konzentration und bei systemischer Anwendung vorzugsweise in einer Einzeldosis von 10 bis 500 mg enthalten und täglich in einer oder mehreren Dosierungen je nach Art und Schwere der Erkrankungen verabreicht werden.

Üblicherweise verwendete pharmazeutisch-technische Hilfsstoffe sind beispielsweise für die lokale Anwendung Alkohole, wie Ethanol, Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol, Polypropylenglykol, Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxianisol oder butyliertes Hydroxitoluol, oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe toxikologisch unbedenklich und mit den verwendeten Wirkstoffen verträglich sind.

### Beispiel 1

### 4-Chlor-3′,5′-dimethyl-4′-[2-(N-pyrrolidinyl)-ethoxy]-chalkonsemifumarat

46,7 g (0,38 Mol) 2,6-Dimethylphenol, 65,0 g (0,38 Mol) N-2(-Chlorethyl)pyrrolidinhydrochlorid, 210,1 g (1,52 Mol) Kaliumcarbonat und 2 g NaJ wurden in 300 ml Ethylmethylketon 48 h unter Rückfluß gekocht. Das Reaktionsgemisch wurde i. Vak. eingeengt, der Rückstand zwischen Wasser und Ether verteilt, die organische Phase abgetrennt, mit Wasser gewaschen, getrocknet und mit überschüssiger etherischer Chlorwasserstoff-Lösung versetzt. Das ausgefallene Produkt wurde aus Essigester/Isopropanol (10/1) umkristallisiert. Man erhielt 63 g N-[2(2,6 Dimethylphenoxy)ethyl]pyrrolidinhydrochlorid.

Zu 10,0 g (40 mmol) des obigen Produkts, in 50 ml Methylenchlorid gelöst, gab man 10,1 g (50 mmol) E-4-Chlorzimtsäurechlorid, gelöst in Methylenchlorid, und anschließend portionsweise 10,7 g (80 mmol) wasserfreies Aluminiumchlorid. Man erwärmte für 1 h auf Rückfluß. Das Reaktionsgemisch wurde auf Eis/Salzsäure gegossen, mit verdünnter Natronlauge alkalisiert, die organische Phase abgetrennt, mit Wasser gewaschen, getrocknet und i. Vak. eingeengt. Der Rückstand wurde in heißem i-Propanol gelöst und mit equimolarer Menge Fumarsäure, gelöst in kochendem i-Propanol, versetzt. Das Gemisch wurde eingeengt und der Rückstand aus Ethanol/H₂O umkristallisiert.

Man erhielt 4,9 g des Produkts. Schmp. 215-217°C.

### Beispiel 2

### {3,5-Dimethyl-4[2-(1-pyrrolidinyl)-ethoxy]-phenyl}-E-2-(1-methyl-2-pyrrolyl)ethenylketon-fumarat

100 g (0,82 Mol) 2,6-Dimethylphenol und 167,4 g (1,64 Mol) Essigsäureanhydrid wurden 4 h unter Rückfluß erwärmt. Das Reaktionsgemisch wurde auf Eis gegossen und mit Methylenchlorid extrahiert. Die organische Phase wurde mit verdünnter Natronlauge gewaschen, getrocknet und i. Vak. eingeengt. Man erhielt 138 g Essigsäure-(2,6-dimethylphenyl)-ester. Zu diesem Produkt gab man portionsweise 120,0 g (0,9 Mol) wasserfreies Aluminiumchlorid, wobei die Temperatur auf 85°C anstieg. Man heizte anschließend für 30 Min. auf 110°C und ließ abkühlen. Der noch dickflüssige Brei wurde auf Eis/Salzsäure gegossen und das ausgefallene Produkt abfiltriert. Durch Umkristallisation aus Methanol/Wasser (1/1) erhielt man 103,6 g des 3,5-Dimethyl-4-hydroxyacetophenons.

39,8 g (0,24 Mol) des obigen Produkts, 32,6 g N-(2-Chlorethyl)pyrrolidin, 66,3 g (0,48 Mol) Kaliumkarbonat und 0,5 g Kryptand-[2.2.2] wurden in 300 ml Acetonitril 2 h unter Rückfluß gekocht. Man filtrierte das Karbonat ab und engte das Filtrat i. Vak. ein. Der Rückstand wurde zwischen Methylenchlorid und verdünnter Natronlauge verteilt, die organische Phase mit Wasser gewaschen, getrocknet und i. Vak. eingeengt. Man erhielt 40,5 g des 3,5-Dimethyl-4-(2-(1-pyrrolidinyl)ethoxy)acetophenons als Rohprodukt, das direkt weiter verarbeitet wurde.

5,2 g (20 mmol) des obigen Produkts und 4,4 g (40 Mol) des 1-Methyl-2-pyrrolcarbaldehyds wurden in 100 ml Methanol gelöst, mit 8 g 50%iger Natronlauge versetzt und für 1 h unter Rückfluß erwärmt. Man engte das Reaktionsgemisch i. Vak. ein, verteilte den Rückstand zwischen Wasser und Methylenchlorid, trocknete die organische Phase und engte sie i. Vak. ein. Der Rückstand wurde in wenig heißem Isopropanol aufgenommen und mit equimolarer isopropanolischer Fumarsäure-Lösung versetzt. Man ließ auskristallisieren und erhielt 7,5 g des Zielproduktes. Schmp. 198-201°C.

Analog Beispiel 2 wurden hergestellt
3. {3,5-Dimethyl-4-[2-(1-pyrrolidinyl)-ethoxy]phenyl}-E-2-[1-(i-propyl)-4-pyrazolyl]-ethenylketon-dihydrochlorid
   Öl
4. {3,5-Dimethyl-4-[2-(1-pyrrolidinyl)-ethoxy]phenyl}-E-2-[3-methyl-2-thienyl)ethenylketon-fumarat
   Schmp. 167-169°C.
5. 3′,5′-Dimethyl-4′-[2-(1-pyrrolidinyl)-ethoxy]-2-trifluormethylchalkon-fumarat
   Schmp. 171-174°C.
6. 2-Chlor-3′,5′-dimethyl-4′-[2-(1-pyrrolidinyl)-ethoxy]-chalkon-hydrochlorid
   Schmp. 181-184°C.
7. 3,5-Dimethyl-4-[2-(1-pyrrolidinyl)-ethoxy]-E-2-(1-indolyl)-ethenylketon-fumarat
   Schmp. 217-219°C.

Aus der Verbindung von Beispiel 5 wurde analog der Hydrierung aus Beispiel 1 hergestellt:
8. 3,5-Dimethyl-4-[2-(1-pyrrolidinyl)-ethoxy]-3′-(2-trifluormethylphenyl)-propiophenon-fumarat
   Schmp. 151°C.

## Patentansprüche

1. p-Hydroxyphenon-Derivate der Formel I 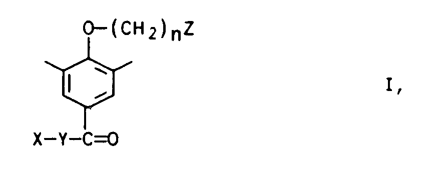 worin
X den Rest 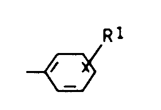 mit R¹ = F, Cl, Br oder Trifluormethyl oder einen Heteroarylrest der Struktur 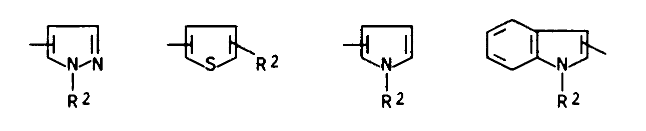 mit R² = C₁-C₄-Alkyl,
Y ein Brückenglied der Formel -CH=CH-, -CH₂CH₂- oder -CH₂-,
n eine der Zahlen 2, 3 oder 4 und
Z eine Aminogruppe der Formel -NR²R³ oder den N-Pyrrolidyl- oder N-Piperidylrest bedeutet, wobei R² und R³ unabhängig voneinander C₁-C₄-Alkyl bedeuten, sowie deren physiologisch verträglichen Salze.

2. Arzneimittel zur topischen Anwendung, das neben üblichen Hilfsstoffen als Wirkstoff 0,0001 bis 1 Gew.% eines p-Hydroxyphenon-Derivates nach Anspruch 1 enthält.

3. Arzneimittel zur systemischen Anwendung, das neben üblichen Hilfsstoffen pro Einzeldosis 10 bis 500 mg eines p-Hydroxyphenon-Derivates nach Anspruch 1 enthält.

4. Antiarrhythmikum der Klasse III nach Vaughan-Williams, das neben üblichen Hilfsstoffen eine wirksame Menge eines p-Hydroxyphenon-Derivates nach Anspruch 1 enthält.

## Claims

1. A p-hydroxy phenone derivative of the formula I 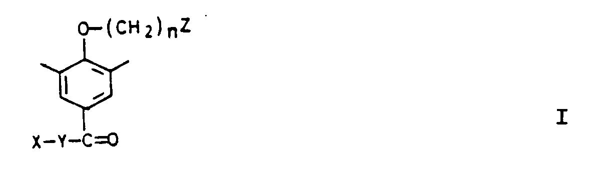 where
X is 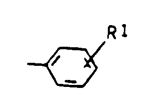 where R¹ = F, Cl, Br or trifluoromethyl or a heteroaryl of the structure 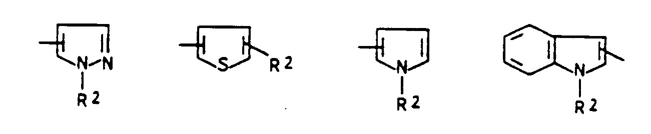 with R² = C₁-C₄-alkyl,
Y is a bridging member of the formula -CH=CH-, -CH₂CH₂- or -CH₂-,
n is one of the numbers 2, 3 or 4 and
Z is an amino group of the formula -NR²R³ or N-pyrrolidyl or N-piperidyl, where R² and R³ are, independently of one another, C₁-C₄-alkyl, as well as the physiologically tolerated salts thereof.

2. A drug for topical administration, which contains as active substance from 0.0001 to 1 % by weight of a p-hydroxy phenome derivative as claimed in claim 1, in addition to conventional aids.

3. A drug for systemic administration, which contains from 10 to 500 mg of a p-hydroxy phenone derivative as claimed in claim 1 per single dose, in addition to conventional aids.

4. An antiarrhythmic of Vaughan-Williams class III, which contains an effective amount of a p-hydroxy phenone derivative as claimed in claim 1, in addition to conventional aids.

## Revendications

1. Dérivés de p-hydroxyphénone de formule I 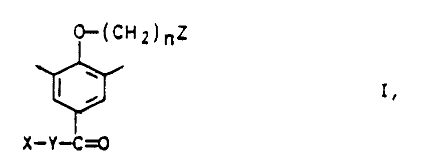 dans laquelle
X représente le reste 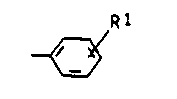 avec R¹ = F, Cl, Br ou trifluorométhyle ou un reste hétéroaryle de la structure 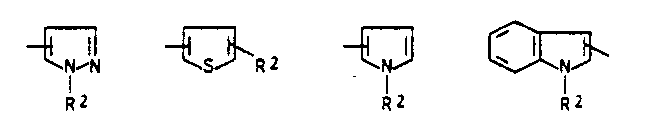 avec R² = alkyle en C1-C4,
Y, un chaînon pontal de la formule -CH=CH, -CH₂CH₂- ou -CH₂-,
n, un des nombres 2, 3 ou 4 et
Z, un groupe amino de la formule -NR²R³ ou le reste N-pyrrolidyle ou N-pipéridyle, R² et R³ représentant indépendamment l'un de l'autre alkyle en C1-C4, ainsi que leurs sels acceptables physiquement.

2. Médicament pour utilisation topique, qui, outre les auxiliaires usuels, contient comme principe actif 0,0001 à 1 % en poids d'un dérivé de p-hydroxyphénone selon la revendication 1.

3. Médicament pour utilisation systémique, qui, outre les auxiliaires usuels, contient par dose unitaire, 10 à 500 mg d'un dérive de p-hydroxyphénone selon la revendication 1.

4. Agent anti-arythmie de la classe III selon Vaughan-Williams, qui, outre des auxiliaires usuels, contient une quantité efficace d'un dérive de p-hydroxyphénone selon la revendication 1.
